Europäisches Patentamt

European Patent Office

Office européen des brevets

(19)

(11) Publication number: **0 286 224 B1**

# EUROPEAN PATENT SPECIFICATION

(12)

(45) Date of publication of patent specification: **25.11.92**

(21) Application number: **88301824.4**

(22) Date of filing: **02.03.88**

(51) Int. Cl.5: **A61K 45/06**, A61K 31/70, A61K 37/66, A61K 31/71, //(A61K45/06,31:70), (A61K37/66,31:70),(A61K31/71, 31:70),(A61K31/70,31:66)

(54) **Treatment of human viral infection by dsRNA combined with viral inhibitors.**

(30) Priority: **23.03.87 US 28823**
**25.11.87 US 125097**

(43) Date of publication of application:
**12.10.88 Bulletin 88/41**

(45) Publication of the grant of the patent:
**25.11.92 Bulletin 92/48**

(84) Designated Contracting States:
**AT BE CH DE ES FR GB GR IT LI LU NL SE**

(56) References cited:
**EP-A- 0 113 162**
**EP-A- 0 196 185**
**EP-A- 0 213 921**

**DIALOG, no. 6160495; M.S.CHAPEKAR et al., pp. 1698-1702, embase no. 86155555***

**DIALOG, no. 6109766; H.R.HUBBELL et al., pp. 359-365, embase no. 86104826***

**JOURNAL OF BIOLOGICAL RESPONSE MODI-**

**FIERS, vol. 6, no. 5, October 1987, Raven Press Ltd, New York, NY (US); H.R.HUBBELL et al., pp. 525-536***

(73) Proprietor: **HEM PHARMACEUTICALS CORP.**
**12280 Wilkins Avenue**
**Rockville, MD 20852(US)**

(72) Inventor: **Carter, William A.**
**1 Jaine Lane**
**Birchrunville, PA(US)**

(74) Representative: **Votier, Sidney David et al**
**CARPMAELS & RANSFORD 43, Bloomsbury Square**
**London WC1A 2RA(GB)**

**Description**

This invention relates to the use of dsRNA in synergistic combination with an inhibitor of reverse transcriptase activity. Pharmaceutical compositions useful in such treatments are also disclosed.

Double-stranded RNAs (dsRNAs), such as poly I•poly C, can act as biological response modifiers eliciting antiviral, antineoplastic and immunomodulatory activities. Among the pleiotropic effects responsible for these biological responses are induction of interferon (IFN) and other cytokines as well as activation of certain INF-induced enzymes including 2,5-oligoadenylate synthetase and a ribosome-associated protein kinase. These properties make dsRNAs attractive candidates for the treatment of infection by human immunodeficiency virus (HIV), the retrovirus responsible for acquired immunodeficiency syndrome (AIDS). In fact, mismatched dsRNA of the form $r(I)_n \cdot r(C_{12-14},U)_n$ or Ampligen® (a registered U.S. trademark of HEM Research, Inc., Rockville, Maryland USA) has a low toxicity profile in humans, is active against HIV infection both in vitro and in vivo. and is currently in large scale, controlled clinical trials of AIDS-related complex (ARC).

The American Foundation for AIDS Research (AmFAR) currently lists over 60 drugs being tested or use in treating ARC and AIDS. This enormous potential of single-agent therapy is compounded by possible synergism in combination therapy. Alternatively, combined therapy has the potential for antagonism as demonstrated in vitro with azidothymidine (AZT) and ribavirin.

For these reasons, I decided to characterize the full potential of mismatched dsRNA in the treatment of ARC and AIDS, HIV used as a prototypic viral pathogen.

I accomplished this by performing in vitro multiple drug analyses using mismatched dsRNA as a core drug in combination with other agents that together encompassed at least five different modes of attack on this virus. These agents included rIFN-alpha A, rIFN-beta Ser 17 and rIFN-$_y$ as cytokines; azidothymidine and phosphonoformate (Foscarnet® (Trade Mark)) as inhibitors of reverse transcription; ribavarin as a putative disrupter of mechanisms governing proper mRNA capping; amphotericin B as a lipid-binding molecule with anti-HIV activity; and castanospermine as an inhibitor of glycoprotein processing (1).

EP-A-0113162 describes a therapeutic composition comprising a therapeutic mixture of a dsRNA and an interferon for use in the treatment of cancer.

EP-A-0213921 describes the use of a dsRNA in the manufacture of a composition for the treatment of HIV including an interferon as an "assisting agent".

EP-A-0196185 describes the use of 3'-azido-3' deoxythymidine or a pharmaceutically acceptable salt as an antiviral agent for use in the treatment of HIV.

According to the present invention, there is provided a product comprising dsRNA and an inhibitor of reverse transcriptase as a combined preparation for simultaneous, separate or sequential use in the treatment of a viral disease by inhibition of viral activity, viral expression or both.

The combination is administered to a patient in an amount sufficient to inhibit viral activity, inhibit viral expression, or both.

Data are here presented verifying the role of dsRNA as a synergistic agent with inhibitors of reverse transcriptase activity in control of viral expression in general and retroviruses in particular utilizing HIV (AIDS virus) as a prototypic human virus associated with chronic debilitating human disease.

Pharmaceutical compositions containing a dsRNA and an inhibitor of reverse transcriptase activity are described and the results of this combination demonstrating synergism reported below.

Accordingly, there is provided a therapeutic composition comprising dsRNA and the inhibitor of reverse transcriptase.

The invention includes therapies for susceptible viral infections other than HIV which have a common mechanism of viral multiplication/pathogenesis, in whole or in part, and accordingly are sensitive to the particular combination employed. Synergism in inhibiting viral activity/expression is unexpectedly seen with reverse transcriptase inhibitors.

The effective treatment of AIDS has obviously become a growing concern among physicians in nearly all countries around the globe. Azidothymidine, the first drug approved in the United States for the treatment of ARC and AIDS, is extremely toxic. This in vivo toxicity is manifested by approximately 30% of patients receiving azidothymidine requiring blood transfusions.

The results of my present experiments suggest that drugs like azidothymidine with high in vivo toxicity can be given at substantially lower, less toxic doses if combined with mismatched dsRNA. Combination therapy may not only reduce the effective dose of a drug required for antiviral activity, thereby reducing its toxicity, but may also improve the absolute antiviral effect as a result of attacking the virus through multiple mechanisms. The pleiotropic activities of mismatched dsRNA together with the synergies reported here suggest that dsRNA in general, and mismatched dsRNA in particular, will be an effective core drug for combination therapy yielding the most effective and least toxic treatment for ARC and AIDS.

These dsRNAs, when administered in combination with inhibitors of reverse transcriptase activity known to cause significant toxicity when administered alone and in quantities effective to address the viral condition, have the additional benefit of permitting the clinician to reduce the amount of the toxic member of the combination without adverse affect on the desired therapeutic results of the treatment.

To demonstrate the synergism of the combinations of this invention, multiple drug effect analyses with mismatched double-stranded RNA (Ampligen® (Trade Mark)) as a core drug were performed to identify other agents and mechanisms through which mismatched dsRNA may potentiate effective therapeutic intervention in human immunodeficiency virus (HIV) infection. Antiviral activities were defined by a microtiter infection assay utilizing MT-2 cells as targets and HTLV-IIIB produced in H9 cells as a virus source. The scope of agents tested ,included azidothymidine and phosphonoformate (Foscarnet® (Trade Mark)) as inhibitors of reverse transcription.

Separately, each drug demonstrated dose-dependent anti-HIV activity and, when used in combination with mismatched dsRNA, demonstrated synergism.

Preferably therefore, the inhibitor of reverse transcriptase is azidothymidine or phosphonoformate, in particular 3'-azido, 3'-deoxythymidine or a pharmaceutically acceptable salt thereof.

The procedures and therapeutic compositions of this invention are intended to include the above-listed agents as exemplary and illustrative of various classes there named.

Preferably the dsRNA is mismatched dsRNA.

By "mismatched dsRNAs" are meant those in which hydrogen bonding (base stacking) between the counterpart strands is relatively intact, i.e., is interrupted on average less than one base pair in every 29 consecutive base residues. The term "mismatched dsRNA" should be understood accordingly.

The dsRNA may be a complex of polyinosinate and a polycytidylate containing a proportion of uracil bases or guanidine bases, e.g., from 1 in 5 to 1 in 30 such bases (poly I $\cdot$ poly ($C_{4-29}$ x > U or G). The dsRNA may be poly I $\cdot$ poly C,U in which the ratio of C to U is about 12-14 to 1, preferably 13:1, and the sedimentation coefficients of poly I and poly C,U are both less than 9 and within 2 units of each other; both are preferably 6.5 to 7.5.

The dsRNA may contain regions of bond breakage.

The mismatched dsRNAs preferred for use in the present invention are based on copolynucleotides selected from poly ($C_n$,G) in which n is an integer having a value of from 4 to 29, and are mismatched analogues of complexes of polyriboinosinic and polyribocytidilic acids, formed by modifying $rI_n \cdot rC_n$ to incorporate unpaired bases (uracil or guanidine) along the polyribocytidylate ($rC_n$) strand. Alternatively, the dsRNA may be derived from poly (I). poly (C) dsRNA by modifying the ribosyl backbone of polyriboinosinic acid ($rI_n$) e.g., by including 2'-0-methyl ribosyl residues.

These mismatched analogues of $rI_n \cdot rC_n$, preferred ones of which are of the general formula $rIn.r\text{-}(C_{11\text{-}14},U)_n$ and $rI_n \cdot r(C_{29},G)$. are described by Carter and Ts'o in U.S. Patents 4,130,641 and 4,024,222. The dsRNAs described therein generally are suitable for use according to the present invention.

Specific examples of mismatched dsRNA for use in the invention include:

poly (I)$\cdot$ poly ($C_4$,U)

poly (I)$\cdot$ poly ($C_7$,U)

poly (I)$\cdot$ poly ($C_{13}$,U)

poly (I)$\cdot$ poly ($C_{22}$,U)

poly (I)$\cdot$ poly ($C_{20}$,G)

poly (I)$\cdot$ poly ($C_{29}$,G) and

poly (I)$\cdot$ poly ($C_p$) 23 G>p

Preferably, the dsRNA is of the general formula $rIn.r(C_{11\text{-}14},U)_n$ and the antiviral agent is 3'-azido, 3'-deoxythymidine.

The amount of mismatched dsRNA administered is preferably sufficient to achieve a peak blood concentration of from 0.1 micrograms per milliliter of dsRNA up to 1000 micrograms per milliliter in the systemic blood circulation immediately following administration distal from the point of infusion. The dsRNA is administered parenterally (intravenously, intramuscularly, subcutaneously), intranasally, rectally or orally when suitably protected against the nucleases of the gastrointestinal tract.

When both agents (a dsRNA and an inhibitor of reverse transcriptase) are administered they may be administered as a mixture, administered separately but simultaneously, or sequentially. The inhibitor of reverse transcriptase is administered in quantities consistent with the product labelling or other directions for use and often in somewhat smaller amounts, frequently substantially reduced, due to the concurrent use of the dsRNA and the synergistic result of the combination.

Administration of a dsRNA and an inhibitor of reverse transcriptase "in combination" includes presentations in which both agents are administered together as a therapeutic mixture, and also procedures in which the two agents are administered separately but simultaneously, e.g., as through separate intravenous lines into the same individual. Administration "in combination" further includes the separate administration of one of the drugs in which one of the drugs is given first followed shortly by the second.

A group of in vitro studies were undertaken to evaluate Ampligen (Trade Mark), a mismatched dsRNA, for combination therapy in the treatment of viral diseases utilizing HIV as a prototypic chronic/subacute human viral pathogen. The materials and methods used are described below.

Cells and Virus - A clone of the HTLV-I-transformed T-cell line MT-2, which exhibits complete cytolysis upon infection with HIV, was used as the target for infections in microtiter assays. Virus was prepared from H9/HTLV-IIIn culture fluids by low speed centrifugation and 0.45 $\mu$M filtration to remove all cells. Viral titers were determined from 50% tissue culture infectious dose (TCID-50) values obtained by endpoint microtitration on MT-2 cells. All cultures were grown and maintained in RPMI-1640 containing 16% heat-inactivated fetal calf serum and 50 $\mu$g gentamicin (Sigma)/ml.

Antivirals - 3'-azido-3' deoxythymidine (AZT, Retrovar® (Trade Mark)), research grade, from Roche Laboratories, and phosphonoformate (Foscarnet® (Trade Mark)) from Astra Alab AB. Mismatched dsRNA (Ampligen® (Trade Mark)) was provided as a lyophilized powder in a salt buffer by HEM Research, Inc., Rockville, Maryland.

Microtiter Infection Assay - Anti-HIV activities were measured in a microtiter infection assay as described (2). Briefly, two-fold, serial dilutions of each drug alone and in fixed-ratio combination with mismatched dsRNA were assayed in triplicate in 96-well microtiter plates. Cytolysis was measured via vital dye (neutral red) uptake by poly L-lysine adherent cells as an endpoint for infection. Cells were incubated in the presence of drug dilutions for 1 hour prior to addition of virus. Cells were infected at a multiplicity of 0.1 so that endpoint cytolysis would be predominantly due to progeny virions synthesized in the presence of the drug. Percent protection was derived from $A_{540}$ values of the dye in test wells relative to the difference in absorption between the cell control and virus control wells using the formula:

$$\% \text{ Protection} = \frac{(test \ minus \ virus)}{(cells \ minus \ virus)}$$

Calculation of Synergy - Combined drug effects were calculated by the multiple drug analysis method of Chou and Talalay using the equation:

$$Cl = \frac{(D_1)}{(Dx)_1} + \frac{(D)_2}{(Dx)_2} + \frac{\alpha(D)_1(D)_2}{(Dx)_1(Dx)_2}$$

where Cl is the combination index, $(Dx)_1$ is the dose of drug 1 required to produce x percent effect alone, and (D). is the dose of drug 1 required to produce the same x percent effect in combination with $(D)_2$. The values of $(Dx)_2$ and $(D)_2$ are similarly derived from drug 2. The value of $\alpha$ is determined from the plot of the dose effect curve using the median effect equation:

$$fa/fu = (D/Dm)^m$$

where fa is the fraction affected by dose D, fu is the unaffected fraction, Dm is the dose required for 50% effect and m is the slope of the dose-effect curve. For mutually exclusive drugs (i.e., similar mode of action), both drugs alone and their mixture give parallel lines in the median effect plot.

Mutually nonexclusive drugs (i.e.. independent mode of action) will give parallel lines in the median effect plot but in mixture will give a concave upward curve. If the agents are mutually exclusive, $\alpha$ is 0, and if they are mutually nonexclusive, $\alpha$ is 1. Values obtained assuming mutual nonexclusiveness will always be slightly greater than mutually exclusive drugs. Cl values of <1 indicate synergy, values >1 indicate antagonism and values equal to 1 indicate additive effects.

Reverse Transcriptase Assay - Reverse transcriptase activities in culture fluids were assayed in polyethylene glycol precipitates as described (3) using poly (A).(dT)$_{15}$ as template primer (Boehringer

Mannheim) and 25 $\mu$Ci [methyl-$^3$H] dTTP (80.1 Ci/mmol, New England Nuclear) per reaction.

Review and analysis of these studies has given the following results and conclusions:

Antiviral Activities - The ability of each drug alone and in combination with mismatched dsRNA to protect target cells from HIV infection is shown in Table 1. Full protection was observed at all concentrations of each drug early in the incubation period immediately following cytolysis in the virus control (no effectors) wells. Virus-induced cytolysis at the lower doses of these drugs occurred one day later and assays were processed again at this time so that dose-dependent relationships could be achieved. The most effective (>10% protection) concentrations of each drug produced greater anti-HIV activity in combination with mismatched dsRNA than when used alone. All drugs were non-toxic to MT-2 cells at the concentrations utilized in these studies.

Table 1

| Mismatched dsRNA ($\mu$g/ml) | Azidothymidine ($\mu$M) | | | | | | | | |
|---|---|---|---|---|---|---|---|---|---|
| | 0 | 0.02 | 0.04 | 0.08 | 0.16 | 0.32 | 0.64 | 1.28 | 2.56 |
| 0 | 0 | 5 | 3 | 5 | 8 | 17 | 30 | 58 | 100 |
| 0.4 | 3 | 2 | | | | | | | |
| 0.8 | 1 | | 1 | | | | | | |
| 1.6 | 4 | | | 9 | | | | | |
| 3.2 | 7 | | | | 23 | | | | |
| 6.4 | 9 | | | | | 65 | | | |
| 12.8 | 10 | | | | | | 84 | | |
| 25.6 | 16 | | | | | | | 94 | |
| 51.2 | 35 | | | | | | | | 100 |

| Mismatched dsRNA ($\mu$g/ml) | Foscarnet ($\mu$g/ml) | | | | | | | | |
|---|---|---|---|---|---|---|---|---|---|
| | 0 | 2 | 4 | 8 | 16 | 32 | 64 | 128 | 256 |
| 0 | 0 | 18 | 42 | 71 | 88 | 95 | 96 | 96 | 99 |
| 0.4 | 12 | 31 | | | | | | | |
| 0.8 | 29 | | 63 | | | | | | |
| 1.6 | 37 | | | 85 | | | | | |
| 3.2 | 52 | | | | 92 | | | | |
| 6.4 | 65 | | | | | 97 | | | |
| 12.8 | 73 | | | | | | 97 | | |
| 25.6 | 79 | | | | | | | 99 | |
| 51.2 | 83 | | | | | | | | 98 |

Multiple Drug Effects - CI values for mismatched dsRNA in dual combination with Foscarnet® (Trade Mark) and azidothymidine at 50% and 95% protections values are given in Table 2. Azidothymidine and phosphonoformate demonstrated synergism with mismatched dsRNA (CI values less than 1) Very little difference was observed if CI values were calculated on the assumption of mutual exclusiveness versus mutual non-exclusiveness for each drug tested.

Table 2

| CI Values for Combined Drug Effects with Mismatched dsRNA as a Core Drug | | | |
|---|---|---|---|
| Drug | CI at Following % Protection Values* | | |
| | 50 | 90 | 95 |
| Azidothymidine | .55 (.56) | .40 (.40) | .37 (.37) |
| Phosphonoformate | .58 (.63) | .65 (.68) | .70 (.72) |

*CI values were calculated from the data in Table 1. Values >1 indicate antagonism, <1 indicate synergism and equal to one indicate an additive effect. CI values calculated assuming mutual exclusiveness are given along with values obtained assuming mutual nonexclusiveness in parentheses.

The synergies observed in this study between mismatched dsRNA and inhibitors of reverse transcriptase suggest that mismatched dsRNA may have a powerful and versatile role as a core drug in combination therapy for ARC and AIDS.

The inhibitors of reverse transcription, have shown to be synergistic with mismatched dsRNA. A broad family of 2',3'-dideoxynucleoside analogues can be metabolized to become potent inhibitors of retroviral reverse transcriptase. For example, R. Yarchoan and S. Broder (New England Journal of Medicine. February 26, 1987, volume 316, pages 557-564) describe two dideoxynucleosides designated 3'-azido-3'deoxythymidine (AZT) and 2',3'-dideoxycytidine (DDC) which are analogues of the naturally utilized thymidine and 2'-deoxycytidine, respectively. See also U.S. Patent No. 4,724,232 describing the use of AZT in the treatment of ARC and AIDS in amounts of from 5 to 250 mg per kilogram body weight of recipient per day. There is evidence that phosphorylated nucleosides inhibit retroviruses by acting as chain terminators such that the viral reverse transcriptase is fooled into adding these analogues to the growing chain of DNA; this blocks further formation of the 5'-3' phosphodiester bond thereby resulting in premature DNA chain termination. Similar effects may be seen with purine analogues such as 2',3'-dideoxyadenosine. Unfortunately, these various pyrimidine and purine analogues can also inhibit various normal cell enzymes, such as DNA polymerase alpha found in bone marrow and other organs. These inhibitions of normal cell functions thus lead to various profound toxicities. For example, chronic administration of AZT causes severe anaemia and leucopenia in more than 50% of cases; accordingly, many AZT treated patients require regular blood transfusions and may actually succumb to the side-effects of bone marrow damage, already weakened by their prior retrovirus infection. The two inhibitors used in this study were azidothymidine and Foscarnet® (Trade Mark).

Azidothymidine, a thymidine analog, becomes phosphorylated intracellularly and is incorporated into nascent DNA where it causes premature chain termination. Phosphorylated azidothymidine is utilized by reverse transcriptase 100 times more effectively than by cellular DNA polymerases, thus allowing a seemingly large window of selectivity.

Phosphonoformate, (Foscarnet® (Trade Mark)) another inhibitor of reverse transcription, has strong anti-HIV activity in vitro in addition to selectively inhibiting influenza virus RNA polymerase and herpes virus DNA polymerase. Both of these drugs demonstrated potent, selective inhibition of HIV in the microtiter infection assay. Their observed synergism with mismatched dsRNA suggests that such synergism may also be observed with other inhibitors of reverse transcription.

dsRNAs in general and Ampligen® (Trade Mark) in particular can substantially reduce by at least 5-fold the concentration of AZT required for significant virustatic activity in vitro. Moreover, at the higher concentrations of AZT tested, there is a synergistic relationship between the two compounds. Therefore, Ampligen® (Trade Mark) will allow a decreased effective therapeutic dose of AZT in vivo with a concomitant decrease in AZT-associated toxicity.

Since the two drugs act by entirely different modes of action, they will demonstrate in vivo no toxicities other than those associated with each drug alone. Indeed, I observed no evidence of synergistic toxicity clinically even when Ampligen® (Trade Mark) was combined with more closely allied molecules such as the interferons. Moreover, since Ampligen® (Trade Mark) has demonstrated clinically in my studies various immunomodulatory activities in addition to its antiviral properties (both possibly mediated through similar mechanisms such as the 2'-5' oligo A pathway), the use of Ampligen® (Trade Mark) in conjunction with

6

AZT may have pronounced and long-term beneficial effects on the course of HIV infections well beyond that which I now report in vitro.

The importance and specificity of my discovery is no better illustrated than in light of the report on the combination of AZT with another powerful anti-AIDS drug, termed ribavarin, which appeared in Science, March 13, 1987, from Dr. Hirsch's laboratory at Harvard: they report profound antagonism when ribavarin is added to AZT in order to achieve therapeutic potentiation. That is, other drugs, when added to reverse transcriptase inhibitors, actually erode their therapeutic potential rather than enhancing it.

The synergistic combination of dsRNAs and inhibitors of reverse transcriptase are also useful in the treatment of retrovirus-induced cancer tumours.

Table of References

1. Elbein, A.D. (1987) Inhibitors of the Biosynthesis and Processing of N-linked Oligosaccharide Chains. Ann. Rev. Biochem. 56,497-534

2. Montefiori, D.C., Robinson, W.E., Jr., Schuffman, S.S. and Mitchell, W.M. (1987) Evaluation of Antiviral Drugs and Neutralizing Antibodies Against Human Immunodeficiency Virus by a Rapid and Sensitive Microtiter Infection Assay. J. Clin Microbiol. (In press)

3. Poiesz, B.J., Ruscetti, F.W., Gazder, A.F., Bunh, P.A., Minna, J.D. and Gallo, R.C. (1980) Detection and Isolation of Type C Retrovirus Particles From Fresh and Cultured Lymphocytes of a Patient With Cutaneous T-cell Lymphoma. Proc. Natl. Acad. Sci. USA 77, 7415-7419.

**Claims**
**Claims for the following Contracting States : AT, BE, CH, DE, FR, GB, IT, LI, LU, NL, SE**

1. A product comprising dsRNA and an inhibitor of reverse transcriptase as a combined preparation for simultaneous, separate or sequential use in the treatment of a viral disease by inhibition of viral activity, viral expression or both.

2. The product of claim 1, which is a therapeutic composition comprising dsRNA and the inhibitor of reverse transcriptase.

3. The product of claim 1 or 2, in which the inhibitor of reverse transcriptase is azidothymidine or phosphonoformate.

4. The product of claim 3, wherein the inhibitor of reverse transcriptase is 3'-azido, 3'-deoxythymidine or a pharmaceutically acceptable salt thereof.

5. The product of any one of claims 1 to 4, wherein the dsRNA is mismatched dsRNA.

6. The product of claim 5, wherein the dsRNA is of the general formula $rI_n.r(C_{11\text{-}14},U)_n$.

7. The product of claim 5, wherein the dsRNA is of the general formula $rI_n {}^\bullet r(C_{29},G)$.

8. The product of any one of claims 5 to 7, wherein the dsRNA contains regions of bond breakage.

9. The product of claim 5, wherein the dsRNA is of the general formula $rI_n {}^\bullet r(C_{11-14}U)_n$ and the antiviral agent is 3'-azido, 3'-deoxythymidine.

10. The use of dsRNA and an inhibitor of reverse transcriptase in the manufacture of a medicament for the treatment of viral disease.

**Claims for the following Contracting States : ES, GR**

1. A process for the production of a combined preparation for simultaneous, separate or sequential use in the treatment of a viral disease by inhibition of viral activity, viral expression or both comprising admixing dsRNA and an inhibitor of reverse transcriptase.

2. The process of claim 1, wherein the combined preparation is a therapeutic composition comprising dsRNA and the inhibitor of reverse transcriptase.

3. The process of claim 1 or 2, in which the inhibitor of reverse transcriptase is azidothymidine or phosphonoformate.

4. The process of claim 3, wherein the inhibitor of reverse transcriptase is 3'-azido, 3'-deoxythymidine or a pharmaceutically acceptable salt thereof.

5. The process of any one of claims 1 to 4, wherein the dsRNA is mismatched dsRNA.

6. The process of claim 5, wherein the dsRNA is of the general formula $rI_n.r(C_{11\text{-}14},U)_n$.

7. The process of claim 5, wherein the dsRNA is of the general formula $rI_n \bullet r(C_{29},G)$.

8. The process of any one of claims 5 to 7, wherein the dsRNA contains regions of bond breakage.

9. The process of claim 5, wherein the dsRNA is of the general formula $rI_n.r(C_{11-14}U)_n$ and the antiviral agent is 3'-azido, 3'-deoxythymidine.

10. The use of dsRNA and an inhibitor of reverse transcriptase in the manufacture of a medicament for the treatment of viral disease.


**Patentansprüche**

**Patentansprüche für folgende Vertragsstaaten : AT, BE, CH, DE, FR, GB, IT, LI, LU, NL, SE**

1. Produkt, das Doppelstrang-(ds)-RNA und einen Inhibitor für reverse Transkriptase enthält, in Form eines Kombinationspräparates für die gleichzeitige, getrennte oder aufeinanderfolgende Verwendung zur Behandlung einer Viruserkrankung durch Inhibierung von viraler Aktivität, viraler Expression oder beidem.

2. Produkt nach Anspruch 1, das eine therapeutische Zusammensetzung ist und dsRNA und den Inhibitor von reverser Transkriptase enthält.

3. Produkt nach Anspruch 1 oder 2, bei welchem der Inhibitor der reversen Transkriptase Azidothymidin oder Phosphoformat ist.

4. Produkt nach Anspruch 3, bei welchem der Inhibitor der reversen Transkriptase 3'-Azido-3'-deoxythymidin oder ein pharmazeutisch verwendbares Salz desselben ist.

5. Produkt nach einem der Ansprüche 1 bis 4, bei welchem die dsRNA eine mismatched dsRNA ist.

6. Produkt nach Anspruch 5, bei welchem die dsRNA der allgemeinen Formel $rI_n.r(C_{11-14},U)_n$ entspricht.

7. Produkt nach Anspruch 5, bei welchem die dsRNA der allgemeinen Formel $rI_n \bullet r(C_{29},G)_n$ entspricht.

8. Produkt nach einem der Ansprüche 5 bis 7, bei welchem die dsRNA Bindungsbruchbereiche enthält.

9. Produkt nach Anspruch 5, bei welchem die dsRNA der allgemeinen Formel $rI.r(C_{11-14}U)_n$ entspricht und das antivirale Mittel 3'-Azido-3'-deoxythymidin ist.

10. Verwendung von dsRNA und einem Inhibitor der reversen Transkriptase bei der Herstellung eines Arzneimittels zur Behandlung von Viruserkrankungen.


**Patentansprüche für folgende Vertragsstaaten : ES, GR**

1. Verfahren zur Herstellung eines Kombinationspräparates zur gleichzeitigen, getrennten oder aufeinanderfolgenden Verwendung bei der Behandlung einer Viruserkrankung durch Inhibierung der viralen Aktivität, viralen Expression oder von beidem, bei welchem Verfahren Doppelstrang-(ds)-RNA und ein Inhibitor für reverse Tranksriptase gemischt werden.

2.  Verfahren nach Anspruch 1, bei welchem das Kombinationspräparat eine therapeutische Zusammensetzung ist, die dsRNA und den Inhibitor für reverse Transkriptase enthält.

3.  Verfahren nach Anspruch 1 oder 2, bei welchem der Inhibitor der reversen Transkriptase Azidothymidin oder Phosphonoformat ist.

4.  Verfahren nach Anspruch 3, bei welchem der Inhibitor der reversen Transkriptase 3'-Azido-3'-deoxythymidin oder ein pharmazeutisch verwendbares Salz desselben ist.

5.  Verfahren nach einem der Ansprüche 1 bis 4, bei welchem die dsRNA eine mismatched dsRNA ist.

6.  Verfahren nach Anspruch 5, bei welchem die dsRNA der allgemeinen Formel $rI_n.r(C_{11-14},U)_n$ entspricht.

7.  Verfahren nach Anspruch 5, bei welchem die dsRNA der allgemeinen Formel $rI_n{}^{\bullet}r(C_{29},G)_n$ entspricht.

8.  Verfahren nach einem der Ansprüche 5 bis 7, bei welchem die dsRNA Bindungsbruchbereiche enthält.

9.  Verfahren nach Anspruch 5, bei welchem die dsRNA der allgemeinen Formel $rI.r(C_{11-14}U)_n$ entspricht und das antivirale Mittel 3'-Azido-3'-deoxythymidin ist.

10. Verwendung von dsRNA und einem Inhibitor der reversen Transkriptase bei der Herstellung eines Arzneimittels zur Behandlung von Viruserkrankungen.

**Revendications**
**Revendications pour les Etats contractants suivants : AT, BE, CH, DE, FR, GB, IT, LI, LU, NL, SE**

1.  Produit comprenant un ARN double brins et un inhibiteur de transcriptase inverse sous forme de préparation combinée pour utilisation simultanée, séparée ou séquentielle dans le traitement d'une maladie virale par inhibition de l'activité virale, de l'expression virale ou des deux.

2.  Produit selon la revendication 1, qui est une composition thérapeutique comprenant un ARN double brins et l'inhibiteur de transcriptase inverse.

3.  Produit selon la revendication 1 ou 2, dans lequel l'inhibiteur de transcriptase inverse est une azidothymidine ou un phosphonoformiate.

4.  Produit selon la revendication 3, dans lequel l'inhibiteur de transcriptase inverse est la 3'-azido-3'-désoxythymidine ou un de ses sels pharmaceutiquement acceptables.

5.  Produit selon l'une quelconque des revendications 1 à 4, dans lequel l'ARN double brins est un ARN double brins anti-sens.

6.  Produit selon la revendication 5, dans lequel l'ARN double brins répond à la formule générale $rI_n.r(C_{11-14},U)_n$.

7.  Produit selon la revendication 5, dans lequel l'ARN double brins répond à la formule générale $rI_n.r(C_{29},G)$.

8.  Produit selon l'une quelconque des revendications 5 à 7, dans lequel l'ARN double brins contient des régions de rupture de liaison.

9.  Produit selon la revendication 5, dans lequel l'ARN double brins répond à la formule générale $rI.r(C_{11-14}U)_n$ et l'agent anti-viral est la 3'-azido-3'-désoxythymidine.

10. Utilisation d'un ARN double brins et d'un inhibiteur de transcriptase inverse dans la fabrication d'un médicament pour le traitement d'une maladie virale.

**Revendications pour les Etats contractants suivants : ES, GR**

1. Procédé de production d'une préparation combinée pour utilisation simultanée, séparée ou séquentielle dans le traitement d'une maladie virale par inhibition de l'activité virale, de l'expression virale ou des deux, comprenant le mélange d'ARN double brins et d'un inhibiteur de transcriptase inverse.

2. Procédé selon la revendication 1, dans lequel la préparation combinée est une composition thérapeutique comprenant un ARN double brins et l'inhibiteur de transcriptase inverse.

3. Procédé selon la revendication 1 ou 2, dans lequel l'inhibiteur de transcriptase inverse est une azidothymidine ou un phosphonoformiate.

4. Procédé selon la revendication 3, dans lequel l'inhibiteur de transcriptase inverse est la 3'-azido-3'-désoxythymidine ou un de ses sels pharmaceutiquement acceptables.

5. Procédé selon l'une quelconque des revendications 1 à 4, dans lequel l'ARN double brins est un ARN double brins anti-sens.

6. Produit selon la revendication 5, dans lequel l'ARN double brins répond à la formule générale $rI_n.r\text{-}(C_{11-14},U)_n$.

7. Produit selon la revendication 5, dans lequel l'ARN double brins répond à la formule générale $rI_n.r\text{-}(C_{29},G)$.

8. Produit selon l'une quelconque des revendications 5 à 7, dans lequel l'ARN double brins contient des régions de rupture de liaison.

9. Produit selon la revendication 5, dans lequel l'ARN double brins répond à la formule générale $rI.r\text{-}(C_{11-14}U)_n$ et l'agent anti-viral est la 3'-azido-3'-désoxythymidine.

10. Utilisation d'un ARN double brins et d'un inhibiteur de transcriptase inverse dans la fabrication d'un médicament pour le traitement d'une maladie virale.